Europäisches Patentamt

European Patent Office

Office européen des brevets

(11) Publication number: **0 458 288 A2**

# (12) EUROPEAN PATENT APPLICATION

(21) Application number: 91108219.6

(22) Date of filing: 22.05.91

(51) Int. Cl.⁵: **C12M 1/40**, C12Q 1/26

(30) Priority: 22.05.90 JP 133497/90

(43) Date of publication of application:
27.11.91 Bulletin 91/48

(84) Designated Contracting States:
DE GB

(71) Applicant: KANZAKI PAPER MANUFACTURING
CO., LTD.
7, Kandaogawamachi 3-chome Chiyoda-ku
Tokyo 101(JP)

(72) Inventor: Hayashi, Ryuzo
7-26, Hishiyanishi 5-chome
Higashiosaka-shi, Osaka, 577(JP)
Inventor: Kariyone, Akio
8-36, Tojiinkitamachi, Kita-ku
Kyoto-shi, Kyoto, 603(JP)
Inventor: Hashizume, Yoshio
Puresuteiji Nishiakashi 702, 1051-1 Fujie
Akashi-shi, Hyogo-ken, 673(JP)

(74) Representative: Patentanwälte Beetz sen. -
Beetz jun. Timpe - Siegfried -
Schmitt-Fumian- Mayr
Steinsdorfstrasse 10
W-8000 München 22(DE)

(54) Method and apparatus for measurement of alcohols.

(57) The measurement utilizes the flow type method in measuring an alcohol content, including steps of: introducing a sample to be analyzed together with a buffer solution (1) into a column (5) filled with a carrier on which surface alcohol oxidase is immobilized; detecting, downstream of the column (5), amount of oxygen decreased or amount of hydrogen peroxide produced, wherein the improvement is included for enabling the measurement for a wide range of alcohol content levels. Specifically, azide compound, for example, sodium azide, is added as a reversible inhibitor into the sample, commensurate to possible alcohol content present therein. In apparatus aspect, further to basic conventional components, a device (3) is added for mixing and transferring the buffer solution (1) and the reversible inhibitor solution (17), for which work, two pumps (2, 18) are installed; a pump (2) for transferring the buffer solution (1), and another pump (18) for transferring a solution (17) containing the reversible inhibitor, and a computer (15) is also incorporated to compute feasible conditions for controlling a sum of flow rates from respective pumps (2, 18) to be constant while permitting a change in a flow rate from either pump.

Fig. 3

## BACKGROUND OF THE INVENTION

### 1. Field of the Invention

This invention relates to a method and an apparatus which enable determination of alcohol content cover a wide range with use of immobilized alcohol oxidase.

### 2. Description of the related art

Apparatus for measurement with use of immobilized enzyme has features in simplicity, quickness, specificity to substrate, for example, and has been finding applications in wide fields including clinical analysis, food analysis, environmental analysis.

Review of the development of apparatus for measurement with use of immobilized enzyme teaches that analysis of alcohols, especially analysis of ethanol has met much demand for its further progress in the field of clinical analysis, food analysis, fermentation and environmental analysis, wherein therefore a number of new proposals have been made. As kind of enzymes for use to analysis of alcohols, useful have been alcohol dehydrogenase (EC. 1. 1. 1. 1) and alcohol oxidase (EC. 1. 1. 3. 13). Then, alcohol dehydrogenase demands NAD (nicotine amide adenine dinucleotide) as coenzyme, which leads reagents for analysis to be costly, and the enzyme itself is unstable, which limits the use thereof only in the form of solution.

Accordingly, such an apparatus with immobilized enzyme as conducts amperometric measurements, that is, apparatus for measuring quantitative changes of electroactive substance caused by immobilized enzyme, by means of detecting those changes as variations of current output from the working electrode at a predetermined voltage, has been known. This kind of apparatus is convenient to be made highly sensitive and superior in stability. On this account, various electrodes, apparatus, and processing for that approach have been devised for further development. Representative method of amperometric analyses includes two kinds; use of oxygen electrode and hydrogen peroxide electrode. These two kinds are most popularly applied to the apparatus with use of immobilized enzyme, wherein the method of using the hydrogen peroxide electrode is superior in response speed and S/N ratio. For the reason noted, as enzyme which may be applied to the apparatus with use of hydrogen peroxide electrode, alcohol oxidase is given attention.

However, in the analysis of clinical, foodstuffs, and fermentative fields, samples for analysis have large difference in alcohol content or concentration, which makes it difficult for any analyses to be dealt with use of single immobilized enzyme. The reason is that alcohol oxidase oxidizes a substrate with consuming dissolved oxygen, and therefore, some enhancement of sensitivity to prepare for low concentrations invites, when a sample having a high concentration is charged, such adverse situation as dissolved oxygen is exhausted by the sample, which therefore causes a stop of progress of the enzymatic reaction and as a result, linear relationship between alcohol contents and response data is lost.

In review of such difficulty, conventionally the apparatus has been organized to suit analysis of low alcohol contents, and a sample having a high alcohol content was diluted largely to suit the apparatus, otherwise a plurality of such apparatus were prepared to deal with different content levels. However, a large dilution makes an error in measurement larger, thus undesirable, and preparation of a plurality of apparatus is also undesirable costwise.

## SUMMARY OF THE INVENTION

An object of the invention is to present a method including the use of immobilized alcohol oxidase column which permits analysis or treatment of samples having a wide range of alcohol concentrations, and to present an apparatus which is versatile in carrying out the method as noted with simplicity.

Other objects and advantages will be apparent as the description proceeds in the following.

The inventors have found that activity of alcohol oxidase immobilized on the surface of a carrier is controlled by adding a reversible inhibitor into a buffer solution which flows through an enzyme-immobilized column, without giving any adverse effect on durability and other characteristics of the immobilized alcohol oxidase. And also that adjustment or control in a concentration of the reversible inhibitor permits the analysis of alcohols having a wide range of concentrations with use of single immobilized alcohol oxidase column. Through these discoveries, the present invention is attained.

In essence, this invention comprises a flow type method of measuring alcohol that a sample to be analyzed is introduced into a column incorporating a carrier, on which surface alcohol oxidase is immobilized, together with a buffer solution, and detecting an amount of oxygen decreased or an amount of hydrogen peroxide produced by enzymatic reaction in the column at a position downstream of the column, with feature that a measurable range for the alcohols is regulated by using the buffer solution comprises reversible inhibitor commensurate to a possible alcohol concentration contained in the sample, whereby the range of measurable alcohol content is regulated suitably.

Specifically, in this invention, the reversible inhibitor comprises an azide compound.

Further, this invention comprises an apparatus for flow type measurement for alcohol contents, said apparatus comprises; (a) means for mixing a buffer solution with a reversible inhibitor and transferring the mixed solution, (b) a column incorporating a carrier, on which surface alcohol oxidase is immobilized, and (c) an oxygen electrode or hydrogen peroxide electrode, wherein these elements are arranged from upstream to downstream in this order, additionally comprised is (d) means for setting a mixing ratio between the buffer solution and the reversible inhibitor solution in the mixing and transferring means.

In operation, the reversible inhibitor of this invention has the property of impeding enzymatic reactions, but removal of this agent will reversibly revive enzymatic activities. Preferable are azide compounds, especially in the present invention wherein an azide compound is used in combination with alcohol oxidase. And therein various salt form compounds, for example, sodium azide, potassium azide may be chosen in view of the requirement that azide ions are present in the buffer solution.

Amount of the azide compound to be added into the buffer solution may be determined in view of linearity of the working curve which was previously obtained by measuring standard samples covering a target content range. Generally, at a concentration of an azide compound lying about 10 $\mu$M, some decrease in the activity is observed as compared to none or zero concentration of azide compound and a decrease is maintained up to about 50 mM. Above this level the decrease of the activity does not continue. Therefore, the present invention is feasible by regulating a content of the azide compound within that range noted above commensurate to a possible alcohol concentration in a target sample. Feasibility is obtained from minuscule level to highly concentrated level of alcohol contents.

An azide ion concentration in the buffer solution is preferable in the range from 0.1 mM to 50 mM, and at an amount lying above 1 mM and below 10 mM, appropriate inhibition to the enzyme activity is found.

Decrease in activity of alcohol oxidase due to addition of an azide compound causes conditions wherein the alcohol consumption at a certain content level is lessened, thus lessening an amount of dissolved oxygen consumption. This trend of less consumption of the dissolved oxygen helps to maintain the linearity of the working curve for a wider range up to a higher alcohol content and to avoid the phenomenon of saturation. The trend and phenomenon noted above hold true of the hydrogen peroxide electrode, wherein a certain sensitivity range covers a wider range up to a higher alcohol content. It is assumed that this unique result proceeds from the fact that the immobilized alcohol has a relatively large Michaelis constant against alcohols, but a relatively small value of the Michaelis constant against oxygen, which makes rather narrow or short linearity of the working curve in the absence of azide ions.

Consequently, it is recommended to determine an amount of alcohol oxidase to be immobilized so as to suit a sample requiring highest sensitivity among the samples to be measured.

Advantages brought by addition of an azide compound into a buffer solution are as noted above. As a modification thereto, it is permitted for similar consequence to take the measures that a solution containing a certain content of an azide compound and a buffer solution are prepared separately and then, these two solutions are mixed before entry into an immobilized alcohol oxidase column. Thereby quick change of effective alcohol contents is possible.

It is desirable that detection means for apparatus to measure alcohol contents according to the present invention includes an oxygen or hydrogen peroxide electrode, because there is no defect that mixing the two liquids causes fluctuation in the refractive index, for example, and that thereby quantitative measurements will be affected.

As for a buffer solution, there is no specific requirement, but preferably buffering ability resident at the PH region suitable for the immobilized alcohol oxidase. Preferable is phosphate or tris buffer solution which has stable or steady buffering ability in neighborhood of neutral pH zone.

In carrying out a target analysis, convenience is afforded by use of an apparatus which includes means for setting a mixing ratio of the two liquids and means for carrying out the mixing ratio and transferring the mixed liquid. Specifically, such means for mixing the two liquids is embodied into a mechanism which includes a device for measuring a liquid and a stirrer, thus the mixing may be carried out based on the measuring above. However, most preferable mechanism is that two pumps are set to pump each liquid, added with means for merging two flows from the pumps. Wherein it is feasible to adjust constant the sum of amounts from the two pumps, and thereby further feasible is a ready change of the mixing ratio by changing a ratio between delivery amounts of the pumps. Therefore, setting a mixing ratio may be replaced with setting flow rates, hence organization of the apparatus made simple.

Referring further to the means for setting a mixing ratio, it is illustrated as follows: employing a pulse motor as drive and introducing a microcomputer for calculating a mixing ratio, and inputting data so as to cover relationship between a mixing ratio to be obtained and frequency of instruction to be directed to the pulse motors. Thus, computer aided control of the flow ratios is attained. The control similar to the note above may be illustrated by combination of DC servo motor and the controller.

Alcohol oxidase for the immobilized enzyme column may be one which is derived from methanol-assimilating yeast belonging to Candida, Pichia and from Basidiomycetes.

As for the carrier for immobilization and the art for immobilization, conventionally known ones are available. Such carriers include: cellulose, agarose, dextran, chitin, collagen, polymer based on amino acid, polystyrene resin, polyacrylamide, polyvinylalcohol, nylon, ion exchange resin, glass beads, alumina, zirconia, ceramics, sand, carbon, activated carbon, silica gel, molecular sieve, titania, tannin, silicone rubber, acid clay (terra abla), diatomaceous earth, pulverized fire brick. Available art for immobilization includes physical adsorption onto the carrier, and chemical immobilization thereto. Available method for detecting alcohol oxidase reaction includes, for example, charging a colorimetric reagent to react with generated hydrogen peroxide at downstream of the column followed by quantitative determination by spectroscopy, or use of an oxygen electrode or hydrogen peroxide electrode. The later, method of using the electrode, is advantageous in that more accurate measurements can be done without influence by a color of a sample or turbid condition thereof.

Examples of the oxygen electrode are the galvanic cell type and Clark cell type, and an example of the hydrogen peroxide electrode comprises an assembly in which gold or platinum or the like conductive material is used as active or working electrode, on which surface preferably a membrane of selective permeability for hydrogen peroxide is disposed.

As is understood by the description so far, the method or apparatus of the invention will cope with the analysis of alcohol keeping a dilution ratio unchanged and using only one set of apparatus, in a situation not only where high sensitivity is demanded for clinical application, but also where relatively low sensitivity is allowable for alcoholic beverages, for example, Japanese Sake or whisky.

BRIEF DESCRIPTION OF THE DRAWINGS

Other and further objects, features, and advantages of the invention will be more explicit from the following detailed description taken with reference to the drawings wherein:

Fig. 1 is a schematic diagram showing the process and apparatus for analysis of alcohol of flow type related to the example 1.

Fig. 2 is a graphical representation of measurement obtained by the example 1.

Fig. 3 is a schematic diagram showing the process and apparatus for analysis of alcohol of flow type related to the example 2.

Fig. 4 is a schematic flow diagram showing the control of a concentration.

Fig. 5 is a graphical representation of measurement obtained by the example 2.

The description was mainly made with the exemplary case that the enzyme is alcohol oxidase and the inhibitor is aside compound, but the technical thought is true with other inhibitors. This invention can be applied to measurements of methanol, ethanol, n-propanol and others.

These drawings are presented by way of illustrating the invention, and therefore these showings should not be construed as limiting the invention.

DETAILED DESCRIPTION OF THE PREFERRED EMBODIMENTS

Now referring to the drawing, preferred embodiments of the invention are described below.

EXAMPLE 1

(1) Preparation of a Hydrogen Peroxide Electrode

Platinum wire having a diameter of 2 mm was wrapped on its side face with shrinkable Teflon®, and an end of the wire was finished to be smooth by file and emery paper of 1500 grade. This platinum wire was set as working electrode, a platinum plate of 1 cm square was set as counter electrode, a saturated calomel electrode (will be noted SCE below) was set as a reference electrode, and these were subjected to electrolysis; in 0.1 M sulfuric acid, +2.0 V for 10 min. and followed by washing of the platinum wire, and then dried at 40 °C for 10 min. and in turn, immersed in dehydrated toluene containing 10 % γ-aminopropyl triethoxy silane for 1 hr. followed by washing again.

20 mg of Bovine serum albumin (prepared by Sigma Ltd. Fraction V) was dissolved in 1 ml of distilled water, into which solution gultaraldehyde was added to account for 0.2 %, and this mixture of 5 μl was placed quickly on the platinum wire as noted above, and then dried at 40 °C for 15 min. Thus the hydrogen peroxide electrode was attained. (2) Preparation of Immobilized Enzyme Column

150 mg of fire brick (sieved 0.58 to 0.25 mm (30 to 60 meshes)) was dried well and immersed in 1 ml of dehydrated toluene containing 10 % of γ-aminopropyl-triethoxy silane for 1 hr, followed by washing. And the silane coupling agent was washed with toluene and ethanol followed by drying at 120 °C for 2 hrs.

After cooled, 0.5 ml of 5% gultaraldehyde aqueous solution was added and allowed to standing at room temperature for 1 hr. In turn, the carrier was washed well, wherein finally washed with Na phosphate buffer solution having pH = 7 which was then eliminated as much as possible.

The amino silanized carrier thus obtained was charged in the enzyme solution which contains 25 $\mu l$ of alcohol oxidase (prepared by Sigma Ltd., enzyme solution derived from Pichia pastoris) and 225 $\mu l$ of Na phosphate buffer solution having pH = 7, and followed by standing at room temperature for 1 hr. and washed with the buffer solution after the standing. The enzyme immobilized carrier was filled or incorporated into the tube having 3 mm outer diameter, 2 mm inner diameter, and 10 cm length. The tube was made from fluorocarbon resin. (3) Apparatus for Measurement

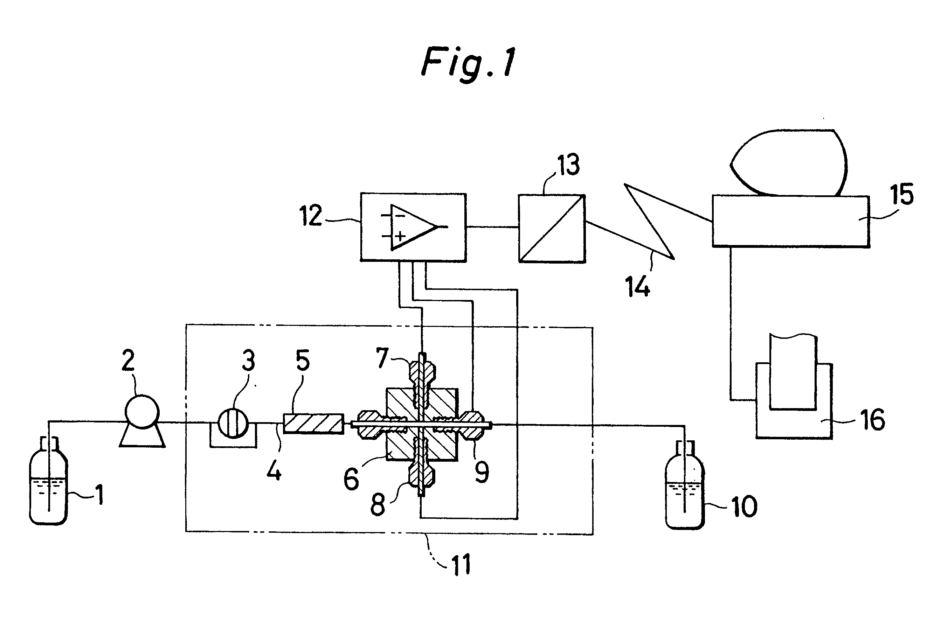

Measurements in this example were made by the flow type apparatus as shown in Fig. 1, which comprises a device for sample injection (Rheodyne Ltd. type 7125 injector) 3, the enzyme immobilized column 5, a measurement cell 6 consisting of the hydrogen peroxide electrode 7 as described above, Ag/AgCl electrode 8 as the reference electrode, and the counter electrode 9 made of stainless steel pipe, in addition to necessary pipings therefor. Wherein a fluororesin pipe 4 of 0.5 mm inner diameter, 0.5 m length was used to connect the injector 3 and the enzyme immobilized column 5, and also the same column 5 and the measurement cell 6. The cell 6 has an inner volume of 40 $\mu l$ wherein the hydrogen peroxide electrode 7 and the Ag/AgCl electrode 8 were disposed in opposite with a passage for the buffer solution inbetween. The hydrogen peroxide electrode 7 was impressed by the potentiostat 12 a voltage of +0.6 V against the Ag/AgCl electrode 8. All of these devices were set inside the thermostat 11, of which inner temperature was kept at 37 ± 0.2 °C. Transfer of the buffer solution 1 was made by a high speed liquid chromatography pump 2 at 1.0 ml/min. rate. The buffer solution used was 100 mM Na phosphate solution having pH = 7.0 charged with sodium azide of different levels as described before.

The buffer solution which has passed the measurement zone was collected into a refuse bottle 10. Measured data was digitized by the A/D convertor 13 followed by sending via the communication cable 14 to the computer 15 for data analysis, of which outcome was printed to the printer 16. (4) Operation for Measurement and Result

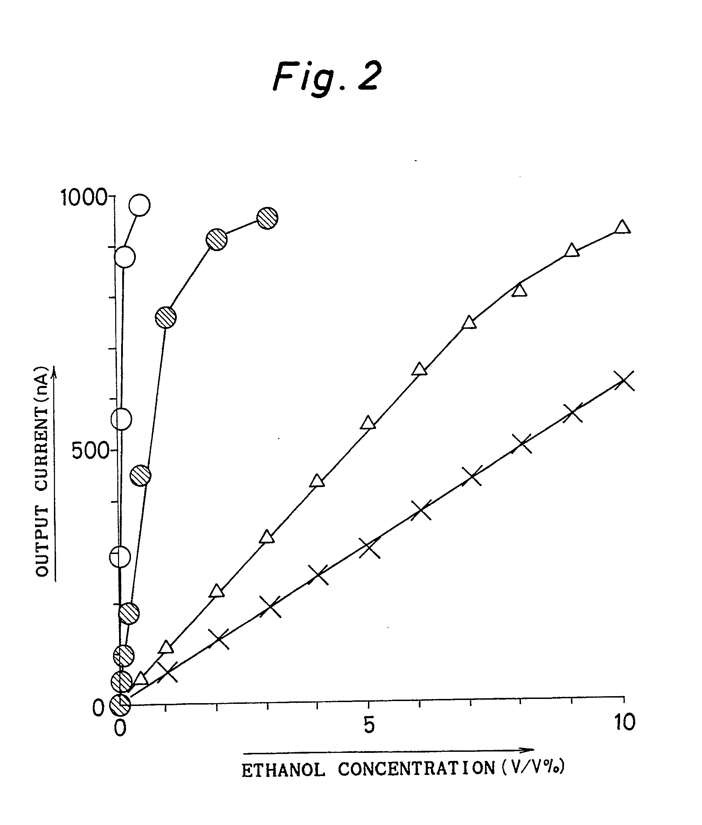

A column immediate after the immobilization was mounted to the measurement apparatus, to which first a phosphate buffer solution having no azide ions was pumped. After the thermostat became equilibrated in temperature, 5 $\mu l$ each of 0 to 10.0 (V/V) % ethanol solution was injected, of which a working curve is shown in Fig. 2 with markings ○ . Then, in the same manner, working curves were obtained with use of buffer solutions containing sodium azide at 0.1 mM, 1 mM, and 10 mM levels, of which outcome was also shown in Fig. 2.

As indicated in Fig. 2, the case containing no azide ions (○)provides a steep inclination with the working curve and justifies the linearity only up to 0.1 %. In contrast, range for the linearity extends: the case containing 0.1 mM (●) does up to 1 %. 1:0 mM (△) up to 6 %. 10 mM (X) up to 10 %. These results teach that measurement can be carried out with presence of decreased amount of azide ions for analysis of lower alcohol content and that the same work can be performed with presence of increased amount of azide ions for analysis of higher alcohol content.

EXAMPLE 2

(1) Preparation of the Hydrogen Peroxide Electrode Prepared in the same manner as in the example 1.
(2) Preparation of the Enzyme Immobilized Column Prepared in the same manner as in the example 1.
(3) Apparatus for Measurement

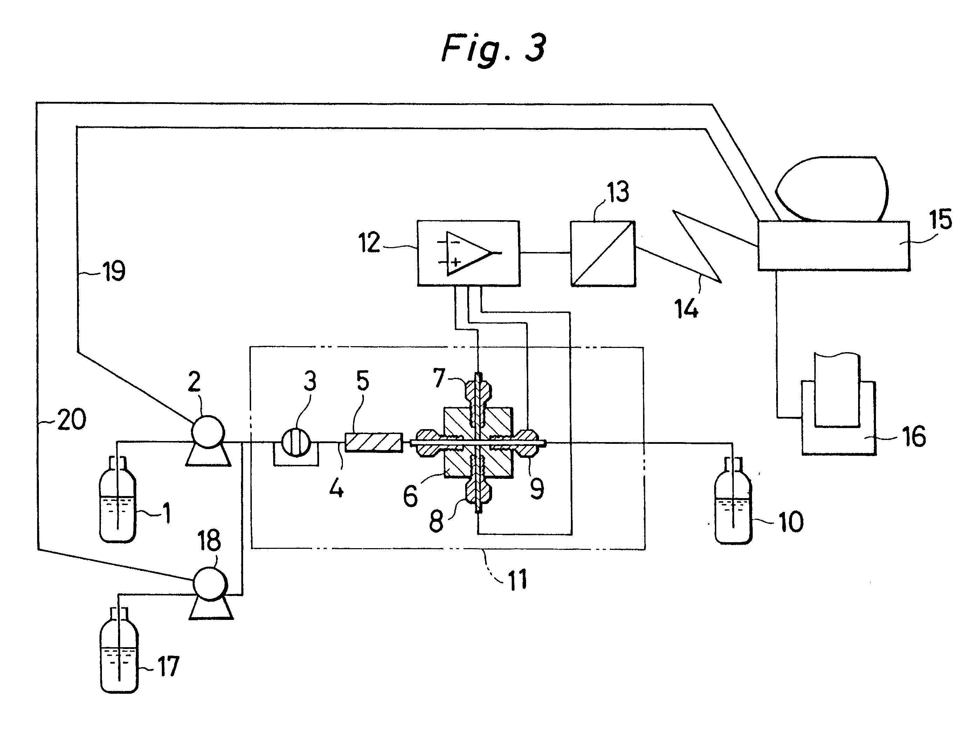

Used was a flow type apparatus as shown in Fig. 3. This apparatus was assembled much with the same features as the one in example 1 in the point of sample injection, column, electrodes involved, except of providing two pumps and two bottles for storing the initial liquid to be sent. Buffer solution 1 is 100 mM Na phosphate buffer having pH = 7.0. Sodium aside solution was stored in the initial bottle 17 which was prepared by adding sodium aside into 100 mM sodium phosphate buffer having pH = 7 so as for the aside to account for 100 mM.

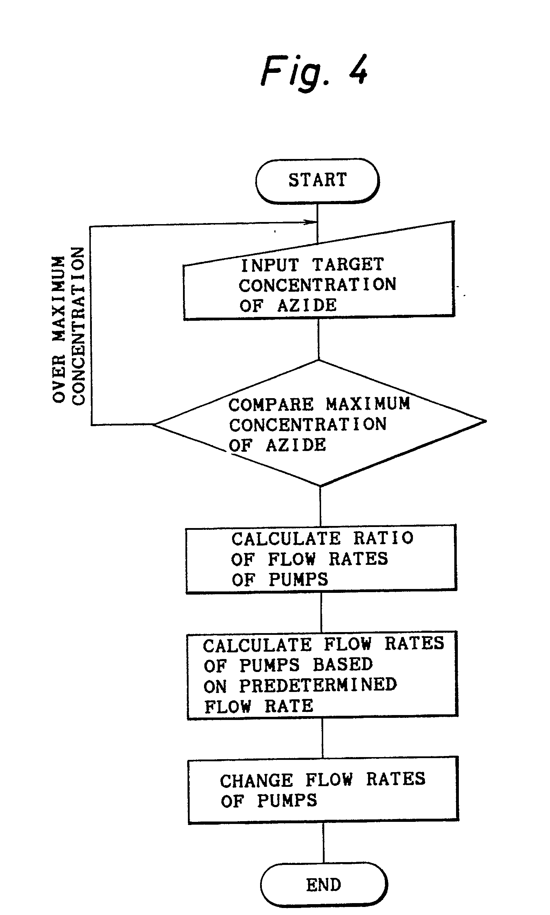

In this illustration, a computer 15 was posted to set a mixing ratio between the buffer solution and the aside-containing solution for means of pumping the mixture, wherein the control of a concentration of the sodium aside would be carried out according to a flow chart as shown in Fig. 4.

Specifically, first a target concentration of sodium aside was input into the computer 15. If the input target concentration was found lower than a concentration stored in the bottle 17 by comparison, a necessary dilution ratio would be computed. Based on the dilution ratio, a ratio between flow rates to be worked by the two pumps would be found. For example, assuming that dilution of 10 times was necessary, it is required that the first pump 2 should have a rate of 9 while the second pump 18 should have a rate of 1, wherein, for the purpose of attaining a flow rate after confluence of 1.0 ml/min., similar to example 1, specific requirements for the two pumps are that the first pump 2 should have 0.9 ml/min. and the second pump 18 should 0.1 ml/min. On these calculations being finished, signals would go, via control cables 19 and 20, to the pumps so as to change the pumping of flow rate.

(4) Operation for Measurement and Result

A column immediate after the immobilization was mounted to the measurement apparatus, to which first a phosphate buffer solution having no azide ions was pumped. After the thermostat became equilibrated in temperature, 5 $\mu$l each of 0 to 10.0 (V/V) % ethanol solution was injected.

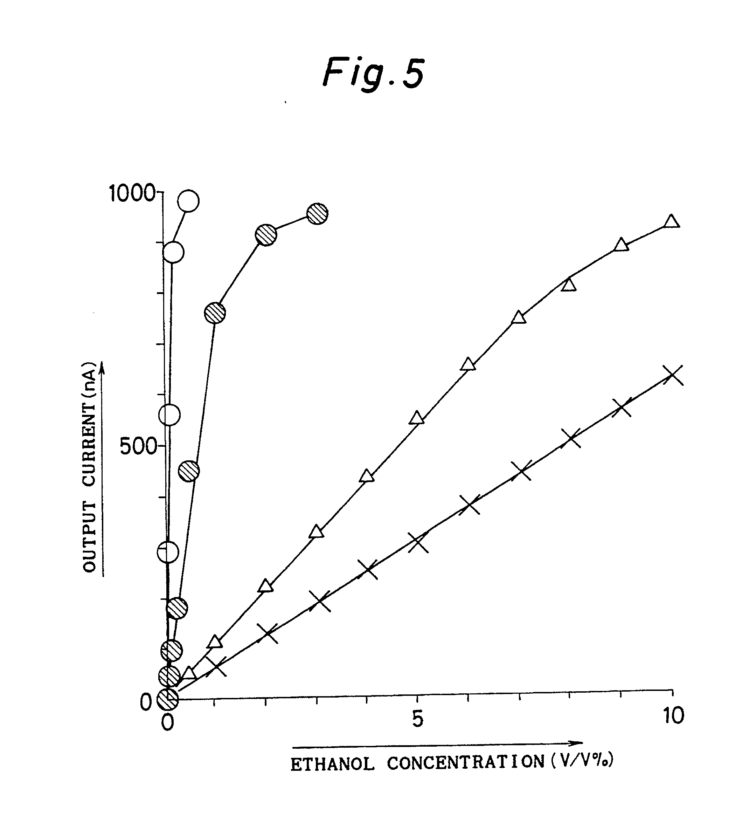

Thereafter, three sodium azide contents were set at 0.1 mM, 1 mM, and 10 mM levels, and the flow or pumping rates were changed by the sequence of the flow map shown in Fig. 4. Working curves were obtained with respective cases applying the appropriate pumping conditions. Consequently obtained were results equivalent to those in example 1 as shown in Fig. 5. Therefore, use of the apparatus in this example expands the feasible range for measurement of alcohol concentration.

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description and all changes which come within the meaning and the range of equivalency of the claims are therefore intended to be embraced therein.

**Claims**

1. A flow type method for measuring an alcohol concentration comprising the steps of:
    introducing a sample to be analyzed together with a buffer solution (1) into a column (5) incorporating a carrier on which surface alcohol oxidase is immobilized;
    detecting, downstream of the column (5), amount of oxygen decreased or amount of hydrogen peroxide produced; and
    wherein the buffer solution (1) contains a reversible inhibitor commensurate to alcohol concentration present in the sample for widening a measurable range of alcohol concentrations.

2. A flow type method for measuring an alcohol concentration as defined in claim 1, wherein the reversible inhibitor is an aside compound.

3. A flow type method for measuring an alcohol concentration as defined in claim 2, wherein the buffer solution (1) contains the azide compound in the range from 0.1 mM to 50 mM.

4. A flow type method for measuring an alcohol concentration as defined in claim 3, wherein the buffer solution (1) contains the azide compound in the range from 1 mM to 10 mM.

5. A flow type method of measuring alcohol comprises steps of:
    a sample to be analyzed is introduced into an immobilized enzyme column (5) which comprises immobilized alcohol oxidase, together with a buffer solution (1), and detecting an amount of oxygen decreased or an amount of hydrogen peroxide produced in the column (5), wherein the buffer solution (1) comprises at least one reversible inhibitor to alcohol oxidase, and said inhibitor is contained at a concentration effective for the inhibitory activity.

6. A flow type apparatus for measuring an alcohol concentration comprising:
    (a) means (3) for mixing and transferring a buffer solution (1) and a reversible inhibitor solution;
    (b) a column (5) incorporating a carrier on which surface alcohol oxidase is immobilized;
    (c) an oxygen electrode or a hydrogen peroxide electrode (7) disposed downstream with respect to the mixing and transferring means (3) and the column (5); and
    (d) means (15) for setting a mixing ratio of the buffer solution (1) with the reversible inhibitor solution in the mixing and transferring means (3).

7. A flow type apparatus for measuring an alcohol concentration as defined in claim 6, wherein the mixing and transferring means comprises:
    a pump (2) for transferring the buffer solution (1); a further pump (18) for transferring a reversible inhibitor solution (17) or buffer solution containing reversible inhibitor;
    piping (4) for merging solutions transferred by the respective pumps (2, 18); and
    means (15) for controlling a sum of flow rates from the respective pumps (2, 18) to be constant while permitting change in a flow rate from the either pump.

8. A flow type apparatus for measuring an alcohol concentration as defined in claim 6, wherein a hydrogen peroxide electrode (7) is provided.

Fig.1

EP 0 458 288 A2

EP 0 458 288 A2

# Fig. 2

# Fig. 3

EP 0 458 288 A2

# *Fig. 4*

START

INPUT TARGET
CONCENTRATION
OF AZIDE

OVER MAXIMUM
CONCENTRATION

COMPARE MAXIMUM
CONCENTRATION
OF AZIDE

CALCULATE RATIO
OF FLOW RATES
OF PUMPS

CALCULATE FLOW RATES
OF PUMPS BASED
ON PREDETERMINED
FLOW RATE

CHANGE FLOW RATES
OF PUMPS

END

# Fig.5